# EUROPEAN PATENT SPECIFICATION

(11) **EP 3 541 205 B1**
(45) Date of publication and mention of the grant of the patent: **22.05.2024**
(21) Application number: 17803873.3
(22) Date of filing: 16.11.2017
(51) Int. Cl.: A23L 33/00, A61K 38/01, A23L 29/281, A23L 33/115, A23L 33/18, A23L 33/185, A23L 33/19, A61P 11/00, A61P 25/00

(54) **NUTRITIONALLY COMPLETE LIQUID COMPOSITIONS FOR USE IN THERAPY OF MALNUTRITION ASSOCIATED WITH COPD, NEUROLOGICAL DISEASES OR DISORDERS AND/OR WOUNDS COMPRISING COLLAGEN HYDROLYSATE**
ERNÄHRUNGSMÄSSIG VOLLSTÄNDIGE FLÜSSIGE ZUSAMMENSETZUNGEN ZUR VERWENDUNG IN DER THERAPIE VON UNTERERNÄHRUNG IM ZUSAMMENHANG MIT CHRONISCH OBSTRUKTIVER LUNGENERKRANKUNG, NEUROLOGISCHEN ERKRANKUNGEN ODER STÖRUNGEN UND/ODER WUNDEN, DIE KOLLAGENHYDROLYSAT ENTHALTEN
COMPOSITIONS LIQUIDES COMPLÈTES SUR LE PLAN NUTRITIONNEL À UTILISER DANS LE TRAITEMENT DE LA MALNUTRITION ASSOCIÉE À LA MALADIE PULMONAIRE OBSTRUCTIVE CHRONIQUE, AUX MALADIES OU TROUBLES NEUROLOGIQUES ET/OU AUX PLAIES, COMPRENANT UN HYDROLYSAT DE COLLAGÈNE

(30) Priority: 16.11.2016 EP 16199103
(43) Date of publication of application: 25.09.2019
(73) Proprietor: Fresenius Kabi Deutschland GmbH, 61352 Bad Homburg (DE)
(72) Inventor: BRITO DE LA FUENTE, Edmundo, 61348 Bad Homburg (DE); PESTANA, Ericka, 61348 Bad Homburg (DE); ASHLEY, Sarah, 61440 Oberursel (DE); MAINOU-SIERRA, José, Maria, 61352 Bad Homburg (DE); MARTINEZ-BOCK, Maria, Fernanda, 61440 Oberursel (DE); REICHART, Stephanie, 61348 Bad Homburg (DE)
(74) Representative: Fresenius Kabi Deutschland GmbH
(86) International application number: PCT/EP2017/079395
(87) International publication number: WO 2018/091566

(56) References cited:
- EP-A1- 2 088 157
- WO-A1-2009/045097
- US-A- 5 733 884
- US-A1- 2007 042 019
- US-A1- 2010 215 631
- US-A1- 2011 008 485
- US-A1- 2016 309 765
- HAYS N P ET AL: "Effects of Whey and Fortified Collagen Hydrolysate Protein Supplements on Nitrogen Balance and Body Composition in Older Women", JOURNAL OF THE AMERICAN DIETETIC ASSOCIATION, THE ASSOCIATION, CHICAGO, IL, US, vol. 109, no. 6, June 2009 (2009-06), pages 1082-1087, XP026132574, ISSN: 0002-8223, DOI: 10.1016/J.JADA.2009.03.003 [retrieved on 2009-05-21]

## Description

### FIELD OF THE INVENTION

The invention relates to PUFA, vitamin E, vitamin D and the protein bound amino acids glycine, arginine and tryptophan as active ingredients for use in the prevention or treatment of malnutrition associated with COPD, for use in the prevention or treatment of malnutrition associated with neurological disease or neurological disorders and for use in prevention or treatment of malnutrition during wound healing, wherein an effective amount of said active ingredients is administered in the form of a nutritionally complete liquid nutritional composition. The invention also relates to the nutritional composition as such.

### BACKGROUND OF THE INVENTION

Chronic obstructive pulmonary disease (COPD) is characterized by a persistent airflow limitation and an abnormal inflammatory response of the airways and is a major cause of mortality.

Due to multiple disease related changes, COPD patients are frequently malnourished. The causes of cachexia in COPD are multifactorial and include tissue hypoxia, ageing, physical exercise, increased resting metabolic rate, chronic inflammatory processes and certain drugs, resulting in net catabolism. There is a clear association between weight loss, body composition, low body mass index (BMI) and low fat free mass index (FFMI) with poor outcome in COPD patients. One of the main factors contributing to malnutrition is a loss of appetite. A pronounced reduction in appetite and decreased oral intake are of central importance in the weight loss that is seen in COPD patients. This is particularly evident during acute exacerbations and may be triggered by difficulties in chewing and swallowing secondary to the extreme shortness of breath.

The consequences of malnutrition in COPD are varied and include changes to body composition, e.g. low body mass index (BMI) and/or low fat free mass index (FFMI), reduced respiratory function, respiratory mechanics as well as physical capacity and ultimately increased morbidity and mortality.

COPD patients are frequently deficient in vitamin D. The consequences of vitamin D deficiency are particularly relevant to COPD patients and include reduced lung function, reduced exercise performance, lower muscle strength and ultimately a higher exacerbation rate.

To ensure COPD patients meet their energy, protein and vitamin D requirements, nutritional compositions, in particular oral nutritional compositions, are needed.

Neurological diseases and disorders are those where the brain and/or spinal cord are affected. They can be subdivided into acute and chronic conditions. Neurological patients are at increased nutritional risk of protein energy malnutrition or micronutrient deficiency when intake is low or metabolic rate is high. The prevalence of malnutrition in patients with neurological diseases or disorders is significant and can increase during hospital admission.

Neurological diseases and disorders can be associated with alterations in resting energy expenditure, leading to hyper or hypo-metabolism due to changes in body composition, altered muscle function, paralysis, rigidity, fasciculation, tremor and other motility disorders. An increase in energy expenditure can increase the need for high calorie and high protein oral nutritional supplements, as it may be more difficult to meet those increased requirements.

Drug therapy can decrease oral intake due to having an unpleasant taste in the mouth or a dry mouth secondary to tablets. Chronic pain is a frequent component of many neurological diseases and disorders. Bowel movements may also be altered, especially with analgesia, which can in turn cause nausea and vomiting.

A decreased intake is one of the main factors leading to malnutrition in patients with neurological diseases or disorders, in particular chronic neurological diseases or disorders. Patients with neurological diseases or disorders, in particular chronic neurological diseases or disorders, frequently suffer from depression, which may also contribute to malnutrition risk. Patients with impaired cognitive function are also at increased risk of malnutrition, due to their impaired ability to shop, buy and prepare food. They may also simply forget to eat without prompting and encouragement.

To ensure patients with neurological diseases or disorders, in particular chronic neurological diseases or disorders, meet their energy, protein and micronutrient requirements, nutritional compositions, in particular oral nutritional compositions, are needed.

Inadequate nutrition slows wound healing, decreases immunocompetence and increases susceptibility to infection. Many aspects of the care and underlying pathologies in patients suffering critical illness can detrimentally influence the normal healing processes of skin and soft tissues.

Malnutrition is recognized as one of the major systemic risk factors for pressure ulcer development. Both poor nutritional intake and poor nutritional status play a role in increasing risk. Protein intake is an important factor affecting wound healing. A deficiency of protein can impair capillary formation, fibroblast proliferation, proteoglycan synthesis, collagen synthesis, and wound remodeling which in turn prevents/delays wound healing. In addition to reduced nutrient availability in the body for energy metabolism, maintenance and repair, malnutrition is also accompanied by losses of fat, decreases in skin resistance, physical weakness, decreased mobility and oedema.

In many cases, increased nutrient requirements cannot be met with food alone, and thus, nutritional compositions, in particular oral nutritional compositions, are needed.

Accordingly, there is a need for systems which can be used in therapy of patients with COPD, neurological diseases or disorders and/or wounds. In particular, there is a need for nutritional compositions which provide sufficient amounts of calories, protein and micronutrients (e.g. vitamin D) in a relatively low volume, as such patients do often not tolerate high or even normal volumes of food and/or nutritional compositions. Preferably, such compositions can be adapted to be nutritionally complete.

Nutritional compositions for use in therapy of COPD or wound healing are known from the prior art, e.g. US 2007/042019 A1, WO 2009/045097 A1, US 2016/309765 A1, US 2011/008485 A1 and US 5 733 884 A.

US 2010/215631 A1 discloses a nutritional composition comprising collagen hydrolysate and milk protein as protein component. The composition is for use in treating inflammatory conditions.

### SUMMARY OF THE INVENTION

The inventors found a system which can be used in the prevention or treatment of malnutrition associated with COPD, in the prevention or treatment of malnutrition associated with neurological disease or neurological disorders and for use in prevention or treatment of malnutrition during wound healing. Said system involves the combined use of PUFA, vitamin E, vitamin D and the protein bound amino acids glycine, arginine and tryptophan as active ingredients.

The corresponding nutritional compositions provide sufficient amounts of calories, protein and micronutrients (e.g. vitamin D) in a relatively low volume, and they can be adapted to be nutritionally complete.

Accordingly, in a first aspect, the present disclosure relates to PUFA, vitamin E, vitamin D and the protein bound amino acids glycine, arginine and tryptophan as active ingredients for use in the prevention or treatment of malnutrition associated with COPD, for use in the prevention or treatment of malnutrition associated with neurological disease or neurological disorders and for use in prevention or treatment of malnutrition during wound healing, wherein an effective amount of said active ingredients is administered in the form of a nutritionally complete liquid nutritional composition comprising a) a lipid component providing 40-50 EN% based on the total energy of the nutritional composition, wherein 12-16 EN% based on the total energy of the nutritional composition is provided by PUFA, b) 4.0-8.0 mg/100mL alpha-tocopherol equivalents (alpha-TE) vitamin E, c) 5.0-12.0 µg/100mL vitamin D, d) a protein component, wherein the protein component consists of collagen hydrolysate as a first protein source and milk protein as a second protein source, wherein collagen hydrolysate is present at a concentration from 70-90 wt% based on the total weight of the protein component and the milk protein is present at a concentration from 10-30 wt% based on the total weight of the protein component and wherein the protein component provides 15-40 EN% based on the total energy of the nutritional composition, wherein the collagen hydrolysate has an average molecular weight M_{w} of from 1000 to 6000 Da, wherein the average molecular weight M_{w} is determined as set forth in the description, e) 2.5-4.5 g/100mL protein bound glycine, f) 0.5-1.5 g/100mL protein bound arginine, g) at least 0.02 g/100mL protein bound tryptophan, and h) wherein the composition has a caloric density of at least 2.0 kcal/ml.

In a second aspect, the present disclosure relates to the nutritional composition as such as defined in claim 13.

Dose units comprising the nutritional composition are disclosed for use according to the claims.

A dosage regime for use in the prevention or treatment of malnutrition associated with COPD, for use in the prevention or treatment of malnutrition associated with neurological disease or neurological disorders and for use in prevention or treatment of inadequate malnutrition during wound healing is disclosed in the appended claims.

### DETAILED DESCRIPTION OF THE INVENTION

### Definitions

"High protein" as used herein refers to nutritional compositions wherein the protein component provides at least 15 EN%, preferably at least 18 EN%, more preferably at least 19 EN%, most preferred at least 20 EN% based on the total energy of the nutritional composition. In preferred embodiments, such nutritional compositions comprise at least 10 wt%, preferably at least 12wt%, more preferably at least 13 wt%, most preferred at least 14 wt% of protein based on the total weight of the nutritional composition. According to the present disclosure, there may be an upper limit to protein content, for example at most 40 EN%, preferably at most 30 EN%, more preferably at most 28 EN%.

"High caloric" as used herein refers to nutritional compositions having a caloric density of at least 2 kcal/mL, preferably at least 2.5 kcal/ml, more preferably at least 2.8 kcal/mL, most preferably at least 3.0 kcal/mL.

"Nutritional composition" herein refers to a synthetically produced food composition. Thus, nutritional compositions are artificial nutritional products obtained by mixing / dissolving bulk ingredients whereby said ingredients are typically provided in solid form (e.g. powders) or liquid from (e.g. oils, water, syrup). The term "nutritional composition" excludes "food", i.e. non-modified natural food products, such as meat, vegetables, fruits in their natural form and conventionally prepared (e.g. cooked) meals or drinks like tea, coffee or juices.

For the present disclosure, "nutritional compositions" are limited to liquid compositions.

"Patient nutrition" as used herein refers to nutrition intended for individuals suffering from a medical condition. Patient nutrition as defined herein excludes the provision of nutrients in the form of conventionally prepared meals ("food"). Patient nutrition therefore only refers to the provision of nutrients in form of nutritional compositions as defined above. Herein, patient nutrition is intended for patients having high caloric needs and high protein needs.

"Nutritionally complete" refers to nutritional compositions suitable as sole source of nutrition. To be nutritionally complete, it is required that a nutritional composition comprises - in addition to the lipid, carbohydrate and protein components - minerals and vitamins. In order to be nutritionally complete, vitamins and minerals should be present in sufficient amounts as known to the man skilled in the art, i.e. in accordance with established nutritional guidelines. The recommended nutrient requirements, in particular with respect to minerals and vitamins, can be found in standard nutritional guidelines such as EU commission directive 1999/21/EC (see tables 2 and 3 hereinbelow). Suitable nutrients according to the present disclosure fulfil the requirements of / are listed in REGULATION (EU) No 609/2013.

"Malnutrition" as used herein refers to one or both of Option I: body mass index (BMI, kg/m2) < 18.5; Option II: the combined finding of unintentional weight loss (mandatory) and at least one of either reduced BMI or a low fat free mass index (FFMI). Weight loss is defined as either > 10% of habitual weight indefinite of time, or >5% over 3 months. Reduced BMI is <20 or <22 kg/m2 in subjects younger and older than 70 years, respectively. Low FFMI is < 15 and < 17 kg/m2 in females and males, respectively.

The term "(nutritional) therapy" as used herein refers to therapy, preferably nutritional therapy.

A composition "consisting of" a number of ingredients or components is to be understood as comprising no other than the named ingredients or components. In case ranges for amounts of ingredients or components are given, the individual amount of all ingredients or components within the composition has of course also to be adapted such that the sum of all amounts of all present ingredients or components adds up to 100 wt%.

"Homogenization" as used herein refers to the process of diminishing the size of the fat globules in a nutritional emulsion. A preferred homogenization process herein comprises two homogenization steps. The first homogenization step may be carried out at a pressure of 100bar and the second homogenization step may be carried out at a pressure of 50 bar. Both steps may be carried out at a temperature of 60-80°C, such as 65-75°C, for example 65 °C.

"UHT-treatment" aims at killing of microorganisms. Preferred UHT treatment may be carried out with a pre-heat treatment at 90°C for 3 min followed by UHT at 139°C for 6 seconds, followed by a (third) homogenization step requiring homogenization at less than 90°C with a pressure that can oscillate between 40-150 bar.

The term "protein bound amino acid" is known to a person skilled in the art. As a person skilled in the art is aware of, a protein is composed of amino acids. Protein bound amino acids are the amino acids which the protein is composed of. In other words, a protein bound amino acid is an amino acid which is bound in a protein. Accordingly, a free amino acid is not a protein bound amino acid. The term "protein" as used in the term "protein bound amino acid" refers to a protein having an average molecular weight (M_{w}) of ≥ 500 Da, preferably ≥ 800 Da, more preferably ≥ 1000 Da. The term "protein" as used in the term "protein bound amino acid" preferably refers to a protein having an average molecular weight (M_{w}) of ≤ 100 kDa, preferably ≤ 80 kDa, more preferably ≤ 60 kDa. The amino acids are bound in collagen hydrolysate or milk protein. One advantage of the use of protein bound amino acids over free amino acids is the improved taste associated therewith.

"Protein component" as used herein refers to the entirety of ingredients of the nutritional composition declarable as "protein".

"Hydrolysed collagen" or "collagen hydrolysate" as used herein refers to low molecular weight collagen obtainable by hydrolysis of collagen by procedures known to the skilled artisan. Hydrolysed collagen herein have an average molecular weight M_{w} of from 1000 Da to 6000 Da, preferably 1000 to 3000 Da. Methods for determining the average molecular weight are well known in the art. An exemplary method is given hereinbelow.

"Lipid component" as used herein refers to the entirety of ingredients of the nutritional composition declarable as "fat". The term "PUFA" as used herein refers to polyunsaturated fatty acids. The term "omega-6-PUFA" as used herein refers to omega-6-polyunsaturated fatty acids. The term "omega-3-PUFA" as used herein refers to omega-3-polyunsaturated fatty acids. The term "MUFA" as used herein refers to monounsaturated fatty acids. The term "SFA" as used herein refers to saturated fatty acids.

"Carbohydrate component" as used herein refers to the entirety of ingredients of the nutritional composition declarable as "carbohydrate".

"EN%" refers to the contribution of a certain component or of a specific ingredient to the total nutritional energy of an edible composition, e.g. the nutritional composition.

"Ready-to-use" refers to the final form of the nutritional composition as administered to a patient. Typically, the nutritional compositions herein are pre-packed in a ready to use format, i.e. sold in separately packed dose units that do not require any further dilution etc..

If not specified otherwise, the expression "/100mL" as used herein means "per 100 mL of the nutritional composition". For example, if not specified otherwise, the expression "g/100mL" as used herein refers to "g per 100 mL of the nutritional composition", the expression "mg/100mL" as used herein refers to "mg per 100 mL of the nutritional composition", and the expression "µg/100mL" as used herein refers to "µg per 100 mL of the nutritional composition", etc..

### Nutritional compositions

The nutritional compositions herein are nutritionally complete and comprise nutrients in predetermined and controllable amounts. A nutritional composition according to the present disclosure comprises a protein component, a lipid component, vitamins and minerals. Such a nutritional composition further comprises a carbohydrate component. Optionally, such a nutritional composition may further comprise dietary fibres and/or further ingredients known as food additives. In particularly preferred embodiments, such a nutritional composition further comprises water.

The nutritional compositions have a high caloric density and a high protein content.

The nutritional compositions have a caloric density of at least 2.0 kcal/mL, preferably at least 2.5 kcal/mL, more preferably at least 2.6 kcal/mL, even more preferably at least 2.8 kcal/mL, even more preferably at least 3.0 kcal/mL, even more preferably at least 3.1 kcal/mL. Typically, the nutritional compositions have a caloric density of at most 5.0 kcal/mL, preferably at most 4.0 kcal/mL, more preferably at most 3.8 kcal/mL, even more preferably at most 3.6 kcal/mL, even more preferably at most 3.4 kcal/mL, even more preferably at most 3.3 kcal/mL. Preferably, the nutritional compositions have a caloric density of 2.0-5.0 kcal/mL, preferably 2.5-4.0 kcal/mL, more preferably 2.6-3.8 kcal/mL, even more preferably 2.8-3.6 kcal/mL, even more preferably 3.0-3.4 kcal/mL, even more preferably 3.1-3.3 kcal/mL. Particular preference is also given to nutritional compositions having a caloric density of 3.0-4.0 kcal/mL.

The nutritional compositions herein may comprise a protein component, a lipid component, a carbohydrate component, wherein
a. the protein component provides 15-40 EN%, preferably 18-40 EN%, more preferably 19-40 EN% based on the total energy of the nutritional composition;
b. the lipid component provides 40-50 EN% based on the total energy of the nutritional composition, wherein 12-16 EN% based on the total energy of the nutritional composition is provided by PUFA;
c. the carbohydrate component provides at least 20 EN% based on the total energy of the nutritional composition.

Preferably, the nutritional compositions according to the present disclosure comprise a protein component, a lipid component, a carbohydrate component, wherein
a. the protein component provides 15-25 EN%, preferably 18-22 EN%, more preferably 19-21 EN% based on the total energy of the nutritional composition;
b. the lipid component provides 40-50 EN%, preferably 43-47 EN%, more preferably 44-46 EN% based on the total energy of the nutritional composition; wherein 12-16 EN% based on the total energy of the nutritional composition is provided by PUFA, and
c. the carbohydrate component provides 30-40 EN%, preferably 33-37 EN%, more preferably 34-36 EN% based on the total energy of the nutritional composition.

In preferred embodiments, the nutritional compositions herein comprise 40-60 wt%, preferably 45-55 wt% of water based on the total weight of the nutritional composition. In preferred embodiments, the nutritional compositions herein comprise 45-65 vol%, preferably 50-60 vol% of water based on the total volume of the nutritional composition.

The nutritional compositions are liquid. In preferred embodiments, the nutritional composition is an emulsion. In particularly preferred embodiments, the nutritional composition is an oil-in-water (O/W) emulsion.

The nutritional composition of the present disclosure is nutritionally complete.

Preferably, the nutritional composition of the present disclosure is a ready to use nutritional composition.

The nutritional composition of the present disclosure is administered enterally, preferably orally.

The nutritional composition of the invention shows good tolerability and palatability resulting in excellent compliance. Good compliance is important because it helps to increase a patient's total protein and energy intake.

In preferred embodiments, the nutritional composition herein comprises the following components in the following amounts:

| | Embodiment I | Embodiment II |
|---|---|---|
| Protein: | 19-21 EN% | 20 EN% |
| Collagen hydrolysate 80% | | 16 g/100mL |
| Milk protein 20% | | |
| glycine* | 3.2-3.7 g/100mL | 3.42 g/100mL |
| arginine* | 0.9-1.2 g/100mL | 1.05 g/100mL |
| tryptophan* | 0.04-0.07 g/100mL | 0.05 g/100mL |
| proline* | 2.2-2.6 g/100mL | 2.38 g/100mL |
| cysteine* | 0.02-0.05 g/100mL | 0.03 g/100mL |
| Fat: | 44-46 EN% | 45 EN% |
| Rapeseed oil | | 16 g/100mL |
| of which SFA | 2-4 EN% | 3 EN% |
| | | 1.1 g/100mL |
| of which MUFA | 26-30 EN% | 28 EN% |
| | | 9.9 g/100mL |
| of which PUFA | 13-15 EN% | 14 EN% |
| | | 5.0 g/100mL |
| CHO | 34-36 EN% | 35 EN% |
| | | 28 g/100mL |
| Caloric density | 3.1-3.3 kcal/mL, preferably 3.2 kcal/mL | 3.2 kcal/mL |
| Water | 50-60 mL/100mL | 56 mL/100mL |
| FSMP balanced ** | yes | yes |
| Vitamin D3 | 7.5-8.5 µg/100mL | 8 µg/100mL |
| Vitamin E | 5.5-6.0 mg/100mL (alpha- tocopherol equivalents (alpha-TE)) | 5.67 mg/100mL (alpha- tocopherol equivalents (alpha-TE)) |

| | | |
|---|---|---|
| * Bound in collagen hydrolysate or milk protein ** Nutritionally complete in vitamins and minerals | | |

In more preferred embodiments, the nutritional composition herein comprises the following components in the following amounts:

| | Embodiment III | Embodiment IV |
|---|---|---|
| Protein: | 19-21 EN% | 20 EN% |
| Collagen hydrolysate 80% | | 16 g/100mL |
| Milk protein 20% | | |
| glycine* | 3.2-3.7 g/100mL | 3.42 g/100mL |
| arginine* | 0.9-1.2 g/100mL | 1.05 g/100mL |
| tryptophan* | 0.04-0.07 g/100mL | 0.05 g/100mL |
| proline* | 2.2-2.6 g/100mL | 2.38 g/100mL |
| cysteine* | 0.02-0.05 g/100mL | 0.03 g/100mL |
| Fat: | 44-46 EN% | 45 EN% |
| Rapeseed oil | | 16 g/100mL |
| of which SFA | 2-4 EN% | 3 EN% |
| | | 1.1 g/100mL |
| of which MUFA | 26-30 EN% | 28 EN% |
| | | 9.9 g/100mL |
| of which PUFA | 13-15 EN% | 14 EN% |
| | | 5.0 g/100mL |
| CHO | 34-36 EN% | 35 EN% |
| | | 28 g/100mL |
| Caloric density | 3.1-3.3 kcal/mL, preferably 3.2 kcal/mL | 3.2 kcal/mL |
| Water | 50-60 mL/100mL | 56 mL/100mL |
| FSMP balanced ** | yes | yes |
| Calcium | 150-175 mg/100mL | 160 mg/100mL |
| Zinc | 3.0-4.0 mg/100mL | 3.5 mg/100mL |
| Copper | 480-540 µg/100mL | 512 µg/100mL |
| Selenium | 20-25 µg/100mL | 23 µg/100mL |
| Vitamin D3 | 7.5-8.5 µg/100mL | 8 µg/100mL |
| Vitamin E | 5.5-6.0 mg/100mL (alpha- tocopherol equivalents (alpha-TE)) | 5.67 mg/100mL (alpha- tocopherol equivalents (alpha-TE)) |
| Vitamin B6 | 0.55-0.62 mg/100mL | 0.58 mg/100mL |
| Vitamin B12 | 1.0-1.2 µg/100mL | 1.1 µg/100mL |
| Folic acid | 60-75 µg/100mL | 67.2 µg/100mL |
| Vitamin C | 35-50 mg/100mL | 41.6 mg/100mL |

| | | |
|---|---|---|
| * Bound in collagen hydrolysate or milk protein ** Nutritionally complete in vitamins and minerals | | |

In even more preferred embodiments, the nutritional composition herein comprises the following components in the following amounts:

| | Embodiment V | Embodiment VI |
|---|---|---|
| Protein: | 19-21 EN% | 20 EN% |
| Collagen hydrolysate 80% | | 16 g/100mL |
| Milk protein 20% | | |
| glycine* | 3.2-3.7 g/100mL | 3.42 g/100mL |
| arginine* | 0.9-1.2 g/100mL | 1.05 g/100mL |
| tryptophan* | 0.04-0.07 g/100mL | 0.05 g/100mL |
| proline* | 2.2-2.6 g/100mL | 2.38 g/100mL |
| cysteine* | 0.02-0.05 g/100mL | 0.03 g/100mL |
| Fat: | 44-46 EN% | 45 EN% |
| Rapeseed oil | | 16 g/100mL |
| of which SFA | 2-4 EN% | 3 EN% |
| | | 1.1 g/100mL |
| of which MUFA | 26-30 EN% | 28 EN% |
| | | 9.9 g/100mL |
| of which PUFA | 13-15 EN% | 14 EN% |
| | | 5.0 g/100mL |
| CHO | 34-36 EN% | 35 EN% |
| | | 28 g/100mL |
| Caloric density | 3.1-3.3 kcal/mL, preferably 3.2 kcal/mL | 3.2 kcal/mL |
| Water | 50-60 mL/100mL | 56 mL/100mL |
| FSMP balanced ** | yes | yes |
| Sodium | 100-125 mg/100mL | 112 mg/100mL |
| Potassium | 290-340 mg/100mL | 312 mg/100mL |
| Chloride | 130-160 mg/100mL | 144 mg/100mL |
| Calcium | 150-175 mg/100mL | 160 mg/100mL |
| Magnesium | 35-45 mg/100mL | 40 mg/100mL |
| Phosphorus | 100-125 mg/100mL | 112 mg/100mL |
| Iron | 4.3-5.2 mg/100mL | 4.8 mg/100mL |
| Zinc | 3.0-4.0 mg/100mL | 3.5 mg/100mL |
| Copper | 480-540 µg/100mL | 512 µg/100mL |
| Manganese | 0.9-1.5 mg/100mL | 1.2 mg/100mL |
| Iodide | 40-50 µg/100mL | 44.8 µg/100mL |
| Fluoride | 0.35-0.45 mg/100mL | 0.4 mg/100mL |
| Chromium | 20-25 µg/100mL | 23 µg/100mL |
| Molybdenum | 27-35 µg/100mL | 30.4 µg/100mL |
| Selenium | 20-25 µg/100mL | 23 µg/100mL |
| Vitamin A | 200-250 µg/100mL (RE***) | 224 µg/100mL (RE***) |
| Beta-Carotene | 400-450 µg/100mL | 426 µg/100mL |
| Vitamin D3 | 7.5-8.5 µg/100mL | 8 µg/100mL |
| Vitamin E | 5.5-6.0 mg/100mL (alpha- tocopherol equivalents (alpha-TE)) | 5.67 mg/100mL (alpha- tocopherol equivalents (alpha-TE)) |
| Vitamin K1 | 17-25 µg/100mL | 20.8 µg/100mL |
| Vitamin B 1 | 0.45-0.55 mg/100mL | 0.5 mg/100mL |
| Vitamin B2 | 0.47-0.55 mg/100mL | 0.51 mg/100mL |
| Niacin | 5.0-5.8 mg/100mL | 5.4 mg/100mL |
| Vitamin B6 | 0.55-0.62 mg/100mL | 0.58 mg/100mL |
| Vitamin B12 | 1.0-1.2 µg/100mL | 1.1 µg/100mL |
| Pantothenic acid | 1.80-2.10 mg/100mL | 1.92 mg/100mL |
| Biotin | 13.0-16.0 µg/100mL | 14.4 µg/100mL |
| Folic acid | 60-75 µg/100mL | 67.2 µg/100mL |
| Vitamin C | 35-50 mg/100mL | 41.6 mg/100mL |

| | | |
|---|---|---|
| * Bound in collagen hydrolysate or milk protein ** Nutritionally complete in vitamins and minerals *** Retinol equivalents | | |

### Protein component

The invention involves the use of the protein bound amino acids glycine, arginine and tryptophan. Accordingly, the nutritional composition herein comprises a protein component.

According to the invention, the nutritional composition comprises 2.5-4.5 g/100mL protein bound glycine. Typically, the nutritional composition comprises 3.0-4.0 g/100mL protein bound glycine, preferably 3.2-3.7 g/100mL protein bound glycine. Glycine promotes the synthesis of creatine in the body, which helps to build lean muscle mass and raise energy levels in the muscles by synthesizing muscle protein increasing nitrogen retention.

According to the invention, the nutritional composition comprises 0.5-1.5 g/100mL protein bound arginine. Typically, the nutritional composition comprises 0.8-1.3 g/100mL protein bound arginine, preferably 0.9-1.2 g/100mL protein bound arginine. Arginine promotes creatine synthesis in the body, which supports muscle metabolism by maintaining a healthy nitrogen balance, which helps to increase muscle mass.

According to the invention, the nutritional composition comprises at least 0.02 g/100mL protein bound tryptophan. Typically, the nutritional composition comprises 0.02-0.2 g/100mL protein bound tryptophan, preferably 0.03-0.1 g/100mL protein bound tryptophan, preferably 0.04-0.07 g/100mL protein bound tryptophan. Tryptophan is an indispensable amino acid, which helps to ensure normal protein metabolism.

Optionally, the nutritional composition further comprises proline, preferably 1.5-4.0 g/100mL proline, more preferably 2.0-3.0 g/100mL proline, even more preferably 2.2-2.6 g/100mL proline.

Optionally, the nutritional composition further comprises cysteine, preferably at least 0.01 g/100mL cysteine, more preferably 0.01-0.1 g/100mL cysteine, even more preferably 0.02-0.05 g/100mL cysteine.

The protein component comprises hydrolysed collagen.

The protein component consists of two different protein sources, wherein the first protein source is hydrolysed collagen and wherein the hydrolysed collagen represents 70-90 wt%, preferably 80-85 wt% based on the total weight of the protein component.

Comparing nutritional compositions comprising high amounts of hydrolysed protein, the nutritional compositions comprising hydrolysed collagen (which are according to the invention) should lead to improved patient compliance due to improved rheological and sensorial properties like viscosity, texture and/or taste.

Within the second protein source, proteins having different average molecular weights may be used. Preferred average molecular weights (M_{w}) of the proteins used within the second protein source lie in the range of 20-60kDa. In such a range properties of the nutritional composition can be well balanced in terms of heat stability and/or viscosity.

For example, a second protein source comprising a high amount of a protein having a lower molecular weight will lead to a reduced viscosity of the nutritional composition. Therefore, a preferred second protein source comprises more than 40 wt%, preferably more than 50 wt% of a protein having an average molecular weight of less than 40kDa, such as 20-40 kDa (based on the total weight of the second protein source).

The protein component consists of collagen hydrolysate as the first protein source and milk protein as the second protein source. Such a protein component is particularly suitable as it can be adapted such that it provides an amino acid distribution suitable to meet current international recommendations for daily intake (e.g. when the nutritional compositions of the present disclosure are used as sole source of nutrition), even without addition of free amino acids, di- or tripeptides. Such an exemplary international recommendation has been published by the WHO (Technical Report Series 935, 2007, p. 150). The protein component comprises 70-90 wt% of hydrolysed collagen as the first protein source and 30-10 wt% milk protein as the second protein source, such as 80 wt% hydrolysed collagen with 20 wt% milk protein (each based on the total weight of the protein component).

Nutritional compositions herein typically comprise at least 10 wt%, preferably at least 12 wt%, more preferably at least 13 wt% of protein based on the total weight of the nutritional composition.

Nutritional compositions herein typically comprise at least 14 g, preferably at least 15 g, more preferably at least 15.5 g, most preferably at least 16 g of protein per 100 mL of the nutritional composition. Nutritional compositions herein typically comprise at most 20 g, preferably at most 18 g, more preferably at most 17.0 g of protein per 100 mL of the nutritional composition. Preferably, nutritional compositions herein comprise 14-20 g, more preferably 15-18 g, most preferably 15.5-17.0 g of protein per 100 mL of the nutritional composition.

In preferred embodiments, the protein component provides at least 15 EN%, preferably at least 18 EN%, more preferably at least 19 EN% based on the total energy of the nutritional composition. For example, the protein component provides 15-25 EN%, preferably 18-22 EN%, more preferably 19-21 EN% based on the total energy of the nutritional composition.

Preferably, the protein to water ratio of the present nutritional composition is at least 2.0/10 [g/g], more preferably at least 2.5/10 [g/g].

As described above, the collagen hydrolysate has an average molecular weight M_{w} of from 1000 Da to 6000 Da, preferably 1000 to 3000 Da. Examples are known to a person skilled in the art and commercially available (e.g. via Gelita, Germany). Such hydrolysates and methods for making them are for example described in DE 102010060564 A1, in particular par. [0004]-[0011] and Example 1 with the low molecular weight hydrolysates in par. [0030] being particularly suitable. Of course, other sources than porcine gelatin can be used, with bovine being particularly preferred for the applications herein.

Accordingly, particularly preferred collagen hydrolysates have molecular weight distributions as listed below:

| Mol. weight range | Upper limit |
|---|---|
| > 7500 Da | 10 wt% |
| > 3500 - 7500 Da | 35 wt% |
| > 1500 - 3500 Da | 35 wt% |
| > 500 - 1500 Da | 50 wt% |
| < 500 Da | 15 wt% |

| Mol. weight range | Preferred range | Particularly preferred range | Example A | Example B |
|---|---|---|---|---|
| > 7500 Da | ≤10 wt% | ≤5 wt% | ≤5 wt% | ≤5 wt% |
| > 3500 - 7500 Da | 10-35 wt% | 10 - 20 wt% | 10 - 20 wt% | 12 - 18 wt% |
| > 1500 - 3500 Da | 20-35 wt% | 25 - 32 wt% | 25 - 32 wt% | 25 - 31 wt% |
| > 500 - 1500 Da | 30-50 wt% | 40 - 50 wt% | 40 - 50 wt% | 40 - 46 wt% |
| < 500 Da | ≤15 wt% | ≤15 wt% | ≤15 wt% | 5 -10 wt% |

Preferably, at most 10 wt%, more preferably at most 5 wt% of the collagen hydrolysate has a molecular weight of above 7,500 Da.

Preferably, at most 15 wt%, more preferably at most 5 wt% of the collagen hydrolysate has a molecular weight of below 500 Da.

Thus, particularly preferred collagen hydrolysates are characterized by at least 75 wt%, preferably at least 90 wt% falling into the molecular weight range of 500-7500 Da.

### Lipid component

The nutritional composition herein comprises a lipid component. Said lipid component may comprise one or more lipid sources, such as lipids of animal and/or vegetable origin. Suitable lipid sources may be selected from oil of marine origin vegetable oils and combinations thereof. Preferably, lipid sources may be selected from fish oil, sunflower oil, safflower oil, soy oil, rapeseed oil, canola oil, linseed oil and combinations thereof. Additionally, the lipid component may comprise MCT in oil or fat form providing C6-C12 fatty acids.

In preferred embodiments, the lipid component comprises rapeseed oil and/or canola oil, preferably rapeseed oil, also preferably canola oil.

In one embodiment, the lipid component comprises fish oil. In another embodiment, the lipid component comprises rapeseed oil and fish oil. In another embodiment, the lipid component comprises canola oil and fish oil.

In terms of individual fatty acids, the lipid component typically includes polyunsaturated fatty acids, monounsaturated fatty acids and saturated fatty acids. Suitable fatty acids may be selected from the group consisting of caproic acid (C6:0), caprylic acid (C8:0), capric acid (C10:0), lauric acid (C12:0), myristic acid (C14:0), palmitic acid (C16:0), palmitoleic acid (C16:1w7), stearic acid (C18:0), oleic acid (C18:1w9), linoleic acid (C18:2w6), a-linolenic acid (C18:3w3), eicosapentaenoic acid (C20:5w3), docosahexaenoic acid (C22:6w3) and mixtures thereof. Particularly preferred fatty acids are linoleic acid, alpha-linolenic acid and mixtures thereof.

Fat is a rich source of energy which is invaluable when meeting a patient's nutritional requirements especially in a small volume. It supports the absorption of fat-soluble vitamins, such as vitamins D and E.

In the nutritional composition of the invention, the percentage energy from fat is higher than the current global nutrition society recommendations for an optimal fat supply in the healthy population. Such higher percentage energy from fat is, however, not contraindicated in the malnourished population. In populations with inadequate total energy intake, dietary fats are an important macronutrient to increase energy intake to appropriate levels. The fat content in the present nutritional composition is appropriate for the malnourished population.

PUFA are a concentrated source of energy and have many benefits. For example, replacing SFA by PUFA reduces the risk of coronary heart disease (CHD). The risk of metabolic syndrome components and diabetes can be reduced as well.

In the nutritional composition of the invention, the percentage energy from PUFA is higher than the WHO recommendation. This recommendation is related to low tocopherol (vitamin E) intake. The main risk in a higher total PUFA dietary intake is thought to be an increased risk of lipid peroxidation. The present nutritional composition provides vitamin E in sufficient amounts, which will not only help the patients to meet their vitamin E requirements but may also further reduce the impact or risk of lipid peroxidation. The PUFA content in the present nutritional composition is appropriate for the malnourished population due to the need for a positive energy balance to promote weight gain in a small volume.

According to the invention, the lipid component provides 40-50 EN% based on the total energy of the nutritional composition. Preferably, the lipid component provides 43-47 EN%, more preferably 44-46 EN% based on the total energy of the nutritional composition.

According to the invention, 12-16 EN% based on the total energy of the nutritional composition is provided by PUFA. Preferably, 13-15 EN% based on the total energy of the nutritional composition is provided by PUFA.

In preferred embodiments, 8-12 EN%, preferably 8.5-10.5 EN% based on the total energy of the nutritional composition is provided by omega-6-PUFA. An example of an omega-6-PUFA is linoleic acid. In preferred embodiments, 3-5 EN%, preferably 3.5-5.0 EN% based on the total energy of the nutritional composition is provided by omega-3-PUFA. An example of an omega-3-PUFA is alpha-linolenic acid. Preferably, the ratio [g/g] of omega-6-PUFA to omega-3-PUFA is 1.6-4.0, more preferably 1.7-3.0.

In preferred embodiments, 24-32 EN%, preferably 26-30 EN%, for example 28 EN%, based on the total energy of the nutritional composition is provided by MUFA. In preferred embodiments, 1-5 EN%, preferably 2-4 EN%, for example 3 EN%, based on the total energy of the nutritional composition is provided by SFA.

### Carbohydrate component

The nutritional composition herein comprises a carbohydrate component. Said carbohydrate component may comprise one or more carbohydrate sources. Typical carbohydrate sources may be selected from the group consisting of maltodextrine, glucose syrup, sucrose, fructose, isomaltulose, starch (modified or unmodified), tapioca dextrine, and mixtures thereof.

Typically, the carbohydrate component provides at least 20 EN%, preferably 30-40 EN%, more preferably 33-37 EN%, for example 34-36 EN% based on the total energy of the nutritional composition.

A preferred carbohydrate component comprises glucose syrup and, preferably sucrose. The carbohydrate component may comprise 65-95 wt% of glucose syrup and 5-35 wt% of sucrose based on the total weight of the carbohydrate component. Preferably, the carbohydrate component comprises 60-80 wt% glucose syrup and 20-40 wt% sucrose, for example 65-75 wt% glucose syrup and 25-35 wt% sucrose based on the total weight of the carbohydrate component.

### Vitamins and Minerals

To be regarded as nutritionally complete, nutritional compositions have to comprise vitamins and minerals.

Suitable vitamins to be included in the composition in order to render it nutritionally complete according to the present disclosure are Vitamin A, Vitamin D, Vitamin K, Vitamin C, Thiamin, Riboflavin, Vitamin B6, Niacin, Folic acid, Vitamin B12, Pantothenic acid, Biotin and Vitamin E. An example for rendering a nutritional composition complete in vitamins is given in table 2.

Suitable minerals to be included in the composition in order to render it nutritionally complete according to the present disclosure are Sodium, Chloride, Potassium, Calcium, Phosphorus, Magnesium, Iron, Zinc, Copper, Iodine, Selenium, Manganese, Chromium and Molybdenum. Optionally, Fluoride may be included. An example for rendering a nutritional composition complete in minerals is given in table 3.

According to the invention, the nutritional composition comprises 4.0-8.0 mg/100mL alpha tocopherol equivalents (alpha-TE) vitamin E. Typically, the nutritional composition comprises 4.5-7.0 mg/100mL alpha- tocopherol equivalents (alpha-TE) vitamin E, preferably 5.0-6.5 mg/100mL alpha- tocopherol equivalents (alpha-TE) vitamin E, more preferably 5.2-6.2 mg/100mL alpha- tocopherol equivalents (alpha-TE) vitamin E, even more preferably 5.5-6.0 mg/100mL alpha- tocopherol equivalents (alpha-TE) vitamin E. The term "alpha-TE" used in connection with vitamin E refers to alpha-tocopherol equivalents. Vitamin E helps to further reduce the impact or risk of lipid peroxidation. Furthermore, vitamin E has antioxidant properties and, thus, may protect tissue, for example brain tissue, from oxidative damage.

According to the invention, the nutritional composition comprises 5.0-12.0 µg/100mL vitamin D. Typically, the nutritional composition comprises 6.0-10.5 µg/100mL vitamin D, preferably 7.0-9.5 µg/100mL vitamin D, more preferably 7.2-9.0 µg/100mL vitamin D, even more preferably 7.5-8.5 µg/100mL vitamin D. The term "vitamin D" as used herein preferably refers to vitamin D3. Vitamin D modulates intestinal calcium absorption and calcium release from bone, as well as renal phosphate excretion in order to maintain plasma calcium and phosphate concentrations in a range supporting cellular processes, neuromuscular function, and bone ossification. Above its role in bone metabolism, vitamin D has additional extra-skeletal influences on the cardiovascular system, the endocrine system, the immune system and the nervous system. Vitamin D decreases the risk of developing numerous chronic diseases, e.g. multiple sclerosis, dementia and Alzheimer's disease, diabetes mellitus, chronic inflammatory bowel disease and cancer. Benefits associated with adequate vitamin D levels include improvements in bone health and skeletal muscle function, prevention or reduced risk of falls, fractures and osteoporosis as well as lower mortality. An adequate intake of vitamin D in conjunction with high protein intake helps to increase muscle mass.

The nutritional composition further comprises B vitamins, preferably at least 5 mg/100mL B vitamins, more preferably 6-20 mg/100mL B vitamins, even more preferably 7-12 mg/100mL B vitamins. Examples of B vitamins are vitamin B1, vitamin B2, niacin, vitamin B6, vitamin B12, pantothenic acid, biotin and folic acid. In preferred embodiments, the nutritional composition comprises vitamin B6, vitamin B12 and folic acid. In particularly preferred embodiments, the nutritional composition comprises vitamin B1, vitamin B2, niacin, vitamin B6, vitamin B12, pantothenic acid, biotin and folic acid. B vitamins, in particular vitamin B6, vitamin B12 and folic acid, help to improve cognitive/psychological function and to decrease cardiovascular risk. In addition, B vitamins may help to decrease the risk of developing Alzheimer's disease and dementia. B vitamins act as cofactors for metabolic processes.

The nutritional composition further comprises vitamin B6, preferably 0.3-1.0 mg/100mL vitamin B6, more preferably 0.5-0.7 mg/100mL vitamin B6, even more preferably 0.55-0.62 mg/100mL vitamin B6. Optionally, the nutritional composition further comprises vitamin B12, preferably 0.6-2.0 µg/100mL vitamin B12, more preferably 0.8-1.5 µg/100mL vitamin B12, even more preferably 1.0-1.2 µg/100mL vitamin B12. Optionally, the nutritional composition further comprises folic acid, preferably 40-100 µg/100mL folic acid, more preferably 50-85 µg/100mL folic acid, even more preferably 60-75 µg/100mL folic acid.

The nutritional composition further comprises vitamin C, preferably 20-80 mg/100mL vitamin C, more preferably 30-60 mg/100mL vitamin C, even more preferably 35-50 mg/100mL vitamin C.

The nutritional composition further comprises calcium, preferably 50-300 mg/100mL calcium, more preferably 120-200 mg/100mL calcium, even more preferably 150-175 mg/100mL calcium. Calcium, in particular in combination with vitamin D, helps to improve bone health and to reduce the risk of bone fragility, osteoporosis and bone fracture.

The nutritional composition further comprises zinc, preferably 2.0-5.0 mg/100mL zinc, more preferably 2.5-4.5 mg/100mL zinc, even more preferably 3.0-4.0 mg/100mL zinc.

The nutritional composition further comprises copper, preferably 400-600 µg/100mL copper, more preferably 450-570 µg/100mL copper, even more preferably 480-540 µg/100mL copper. Due to its antioxidant properties, copper may protect tissue, e.g. brain tissue, from oxidative damage.

The nutritional composition further comprises selenium, preferably 10-40 µg/100mL selenium, more preferably 15-30 µg/100mL selenium, even more preferably 20-25 µg/100mL selenium. Due to its antioxidant properties, selenium may protect tissue, e.g. brain tissue, from oxidative damage

In one embodiment, the nutritional composition further comprises vitamin B6, vitamin B12, folic acid, copper and selenium, preferably in the amounts specified above.

In one embodiment, the nutritional composition further comprises vitamin C and zinc, preferably in the amounts specified above.

### Fibre

The nutritional composition herein may comprise ingredients declarable as dietary fibres. Suitable dietary fibres may be selected from the group consisting of cocoa powder, inulin, wheat dextrine, cellulose, microcrystalline cellulose, soy polysaccharides, tapioca dextrine, xanthan, fructooligosaccharides, galactooligosaccharides, at least partially hydrolysed guar gum, acacia gum, pectin, oat fibre, polydextrose, resistant starch, hemicellulose and mixtures thereof.

### Additives

Nutritional compositions optionally comprise food additives. Additives are typically present in total amount of less than 10wt%, 5wt% or even less than 1wt% based on the total weight of the nutritional composition. Exemplary additives are choline, beta-carotene, lutein, lycopene, caffeine, lecithin, taurine, carnitine, myo-inositol, colorants, aroma and mixtures thereof. Aromas may be caramel, vanilla, yoghurt, chocolate, coffee, cappuccino, fruit aromas and the like.

The additives may include stabilisers and emulsifiers. Preferably, the stabilisers are selected from gums and mixtures thereof. For example, microcrystalline cellulose (E460), sodium carboxymethylcellulose (E466), carrageenan (E407), diacteyl tartaric acid ester of glycerides, cellulose gel (cellulose, microcrystalline) can be used. The emulsifiers may be selected from (destilled) monoglycerides such as E471, soy lecithins. For example, stabilizers and emulsifiers are included in the following amounts / ratios monoglycerides (E471, as an emulsifier) 1-5g/L and a stabilizer mixture comprising 0.3-5g/L, soy lecithin 1-5g/L, diacetyl tartaric acid of glycerides (E472, eg. DATEM) 0.1-5 g/L and MCC 1-8g/L.

### Use in therapy

According to the invention, the nutritional composition is for use in the prevention or treatment of malnutrition associated with COPD, for use in the prevention or treatment of malnutrition associated with neurological disease or neurological disorders and for use in prevention or treatment of malnutrition during wound healing. In preferred embodiments, therapy refers to nutritional therapy. In preferred embodiments, the nutritional composition is used as patient nutrition.

In one embodiment, the patients are patients with COPD and/or neurological diseases or disorders. In another embodiment, the patients are patients with COPD and/or wounds. In another embodiment, the patients are patients with neurological diseases or disorders and/or wounds.

In one preferred embodiment, the patients are patients with COPD. In another preferred embodiment, the patients are patients with neurological diseases or disorders. In another preferred embodiment, the patients are patients with wounds.

If not specified otherwise, the term "patient(s)" as used in the context of the present invention refers to patient(s) with COPD, neurological diseases or disorders and/or wounds, preferably patient(s) with COPD and/or neurological diseases or disorders, also preferably patient(s) with COPD and/or wounds, also preferably patient(s) with neurological diseases or disorders and/or wounds, more preferably patient(s) with COPD, also more preferably patient(s) with neurological diseases or disorders, also more preferably patient(s) with wounds.

Preferred patients may have a low body mass index (BMI). For example, preferred patients have a BMI of < 22 kg/m2, preferably < 20 kg/m2, more preferably < 18.5 kg/m2, even more preferably < 17 kg/m2, even more preferably < 16 kg/m2.

Preferred patients have a BMI of < 22 kg/m2, preferably < 20 kg/m2, more preferably < 18.5 kg/m2, even more preferably < 17 kg/m2, if they are 70 years or older, and a BMI of < 20 kg/m2, preferably < 18.5 kg/m2, more preferably < 17 kg/m2, even more preferably < 16 kg/m2, if they are younger than 70 years.

Preferred patients may have a low fat free mass index (FFMI). Preferred patients have an FFMI of < 16 kg/m2, preferably < 15 kg/m2, more preferably < 14 kg/m2, even more preferably < 13 kg/m2, if they are female, and an FFMI of < 18 kg/m2, preferably < 17 kg/m2, more preferably < 16 kg/m2, even more preferably < 15 kg/m2, if they are male.

Particularly preferred patients may have a low body mass index (BMI) and a low fat free mass index (FFMI). Particularly preferred patients have a BMI of < 22 kg/m2, preferably < 20 kg/m2, more preferably < 18.5 kg/m2, even more preferably < 17 kg/m2, if they are 70 years or older, and a BMI of < 20 kg/m2, preferably < 18.5 kg/m2, more preferably < 17 kg/m2, even more preferably < 16 kg/m2, if they are younger than 70 years, and an FFMI of < 16 kg/m2, preferably < 15 kg/m2, more preferably < 14 kg/m2, even more preferably < 13 kg/m2, if they are female, and an FFMI of < 18 kg/m2, preferably < 17 kg/m2, more preferably < 16 kg/m2, even more preferably < 15 kg/m2, if they are male.

The patients can be at any age. Preferably, the patients are 18 years or older. For example, the patients are 25 years or older, or 30 years or older, or 40 years or older, or 50 years or older, or 60 years or older, or 70 years or older. The patients can, for example, be at home or in a nursing home, a hospital or a hospice.

The nutritional composition is for use in the prevention or treatment of malnutrition, preferably protein malnutrition, also preferably protein-energy malnutrition (PEM) as set forth in the appended claims.

In a particular embodiment, the nutritional composition is for use in the prevention or treatment of protein malnutrition or protein-energy malnutrition (PEM) and vitamin D deficiency as set forth in the appended claims.

Particularly preferably, the nutritional composition is for use in the prevention or treatment of protein malnutrition and vitamin D deficiency as set forth in the appended claims. Also particularly preferably, the nutritional composition is for use in the prevention or treatment of protein-energy malnutrition (PEM) and vitamin D deficiency as set forth in the appended claims. [00119] In one preferred embodiment, the nutritional composition is for use in the prevention or treatment of nutrient deficiencies associated with malnutrition, preferably protein malnutrition, also preferably protein-energy malnutrition (PEM) as set forth in the appended claims.

In a particular embodiment, the nutritional composition is for use in the prevention or treatment of nutrient deficiencies associated with protein malnutrition or protein-energy malnutrition (PEM) and vitamin D deficiency as set forth in the appended claims. Particularly preferably, the nutritional composition is for use in the prevention or treatment of nutrient deficiencies associated with protein malnutrition and vitamin D deficiency as set forth in the appended claims. Also particularly preferably, the nutritional composition is for use in the prevention or treatment of nutrient deficiencies associated with protein-energy malnutrition (PEM) and vitamin D deficiency as set forth in the appended claims.

In one preferred embodiment, the nutritional composition is for use in (nutritional) therapy of patients which suffer from anorexia, cachexia and/or sarcopenia according to the appended claims. In another preferred embodiment, the nutritional composition is for use in (nutritional) therapy of patients which suffer from anorexia and/or cachexia according to the appended claims. In another preferred embodiment, the nutritional composition is for use in (nutritional) therapy of patients which suffer from anorexia and/or sarcopenia according to the appended claims. In another preferred embodiment, the nutritional composition is for use in (nutritional) therapy of patients which suffer from cachexia and/or sarcopenia according to the appended claims.

In one embodiment, the nutritional composition is for use in therapy of patients with COPD and/or neurological diseases or disorders according to the appended claims. In another embodiment, the nutritional composition is for use in therapy of patients with COPD and/or wounds according to the appended claims. In another embodiment, the nutritional composition is for use in therapy of patients with neurological diseases or disorders and/or wounds according to the appended claims.

In one preferred embodiment, the nutritional composition is for use in therapy of patients with COPD according to the appended claims.

Sarcopenia frequently occurs in patients with COPD. Accordingly, in a preferred embodiment, the nutritional composition is for use in (nutritional) therapy of patients with COPD which suffer from sarcopenia according to the appended claims.

Anorexia and/or cachexia frequently occur in patients with COPD. Accordingly, in a preferred embodiment, the nutritional composition is for use in (nutritional) therapy of patients with COPD which suffer from anorexia and/or cachexia according to the appended claims. In a particularly preferred embodiment, the nutritional composition is for use in (nutritional) therapy of patients with COPD which suffer from anorexia according to the appended claims. In another particularly preferred embodiment, the nutritional composition is for use in (nutritional) therapy of patients with COPD which suffer from cachexia according to the appended claims.

In one preferred embodiment, the nutritional composition is for use in therapy of patients with neurological diseases or disorders according to the appended claims.

Preferably, the neurological diseases or disorders are chronic neurological diseases or disorders. The neurological diseases or disorders can, for example, also be acute neurological diseases or disorders.

For example, the neurological diseases or disorders can be selected from systemic atrophies primarily affecting the central nervous system, extrapyramidal and movement disorders and demyelinating diseases of the central nervous system. The neurological diseases or disorders can, for example, also be selected from dementia and Alzheimer's disease.

Preferably, the neurological diseases or disorders are selected from Huntington's disease, dementia, Alzheimer's disease, hereditary ataxia, spinal muscular atrophy, motor neuron disease, amyotrophic lateral sclerosis (ALS), Parkinson's disease (PD), progressive supranuclear palsy and multiple sclerosis (MS). More preferably, the neurological diseases or disorders are selected from dementia, amyotrophic lateral sclerosis (ALS), Parkinson's disease (PD) and multiple sclerosis (MS).

In a particularly preferred embodiment, the neurological disease or disorder is dementia. Prevalence of dementia is increasing worldwide as the older population grows in number. It is a devastating condition leading to dependence and a significant increase in mortality. As a clinical syndrome, it is characterized by global cognitive impairment with a decline in memory and at least in one other cognitive domain, such as language, visuospatial or executive functioning. It represents a decline from the previous level of cognitive functioning, and is associated with impairment in functional abilities and, in many cases, behavioral and psychiatric disturbances. The changes in cognition have an impact on the functional status of the individual. The person will go from being independent to becoming frail to finally being immobile and dependent. Indeed, the cognitive changes will render the individual slowly more vulnerable and frail. These functional changes together with the assessment of difficulties in communication and social interaction will determine the severity of the condition. People suffering from dementia are at increased risk of malnutrition due to various nutritional problems. In the early stage of dementia, patients may have taste and smell changes, which is also a common physiological change in older people. As the disease progresses, the nutritional risks increase as executive functioning declines, ability to plan, shop and cook becomes more difficult to complete and assistance is often required from friends, family or carers. This impacts oral intake significantly and, for example, also when combined with age-related sarcopenia and/or reduced mobility, can cause further decline in cognitive impairment, which in turn reduces the patient's ability, increasing the malnutrition risk further.

In another particularly preferred embodiment, the neurological disease or disorder is amyotrophic lateral sclerosis (ALS). Amyotrophic lateral sclerosis (ALS) is the most common adult-onset motor neuron disease. It is a progressive disorder, involving motor neurons in the cerebral cortex, brainstem and spinal cord, presenting with a combination of upper and lower motor neuron signs including mobility issues, dysarthria, dysphagia, expressive dysphasia and general weakness. Adverse nutritional consequences are common.

In another particularly preferred embodiment, the neurological disease or disorder is Parkinson's disease (PD). Parkinson's disease (PD) is one of the most frequent neurological diseases. As the disease progresses, other symptoms become evident, including dysphagia, impaired gastrointestinal motility, fatigue, depression and cognitive impairment. Adverse nutritional consequences are common. Significantly lower body weight is found in patients with PD, and BMI significantly decreases with increasing disease duration. Patients with PD are also likely to have a faster progression of age-related sarcopenia.

In another particularly preferred embodiment, the neurological disease or disorder is multiple sclerosis (MS). Multiple sclerosis (MS) is a demyelinating disorder characterized by inflammation and selective destruction of central nervous system myelin. There are two types: relapsing-remitting or progressive. It is the second most common cause of neurological disability (after trauma) in young adults. Weight loss in these patients with MS can be due to decreased intake, dysphagia and depression. Immobility is also common in MS.

Sarcopenia may occur in patients with neurological diseases or disorders. Accordingly, in a preferred embodiment, the nutritional composition is for use in (nutritional) therapy of patients with neurological diseases or disorders which suffer from sarcopenia according to the appended claims.

Anorexia and/or cachexia may also occur in patients with neurological diseases or disorders. Accordingly, in a preferred embodiment, the nutritional composition is for use in (nutritional) therapy of patients with neurological diseases or disorders which suffer from anorexia and/or cachexia according to the appended claims. In a particularly preferred embodiment, the nutritional composition is for use in (nutritional) therapy of patients with neurological diseases or disorders which suffer from anorexia according to the appended claims. In another particularly preferred embodiment, the nutritional composition is for use in (nutritional) therapy of patients with neurological diseases or disorders which suffer from cachexia according to the appended claims.

In one preferred embodiment, the nutritional composition is for use in therapy of patients with wounds according to the appended claims. This preferably means that the nutritional composition is for use in wound healing in patients with wounds according to the appended claims.

Preferably, the wounds are selected from pressure ulcers and surgical wounds. Particularly preferably, the wounds are pressure ulcers. Also particularly preferably, the wounds are surgical wounds. Surgical wounds are, for example, wounds resulting from orthopaedic surgery.

In a particular embodiment, the nutritional composition is for use in therapy of patients with pressure ulcers according to the appended claims. This preferably means that the nutritional composition is for use in pressure ulcer healing in patients with pressure ulcers according to the appended claims.

In a particular embodiment, the nutritional composition is for use in therapy of patients with surgical wounds according to the appended claims. This preferably means that the nutritional composition is for use in surgical wound healing in patients with surgical wounds according to the appended claims.

Preferably, the nutritional composition to be administered is provided in a dose unit of 100-200mL, more preferably 100-150mL. Also preferably, the nutritional composition to be administered is provided in a dose unit providing 300-500kcal, more preferably 350-450kcal. Also preferably, the nutritional composition to be administered is provided in a dose unit providing 15-30g, more preferably 18-22g of protein. Particularly preferably, the nutritional composition to be administered is provided in a dose unit of 125mL providing 400kcal and 20g of protein.

Preferably, the nutritional composition for administration in 3-8 dose units daily, preferably 5 dose units daily, for example 5 dose units daily each providing 350-450kcal, or for example 5 dose units daily each providing 18-22g of protein. Also preferably, the nutritional composition is for administration in 1-3 dose units daily, preferably 1 or 2 dose units daily, for example 1 or 2 dose units daily each providing 350-450kcal, or for example 1 or 2 dose units daily each providing 18-22g of protein.

In preferred embodiments, the nutritional composition is for administration in a daily dose of 1500-2500kcal or in a daily dose of 75-125g of protein. Such daily doses are particularly suitable for complete nutrition. In other preferred embodiments, the nutritional composition is for administration in a daily dose of 300-900kcal or in a daily dose of 15-45g of protein. Such daily doses are particularly suitable for supplemental nutrition.

### Preparation of nutritional compositions

The nutritional composition can be prepared according to methods known in the art. For example, the nutritional composition can be prepared by mixing and homogenization. The components of the nutritional composition can, for example, be mixed at elevated temperatures, for example at a temperature of 50-90°C. Homogenization can, for example, be carried out at a temperature of < 90°C, preferably 60-65°C, and/or at a pressure of 40-200 bar, preferably 40-110 bar, more preferably 50-100 bar. Homogenization can, for example, involve two steps (e.g., a first step which can be carried out at 90-110 bar, for example 100 bar, and a second step which can be carried out at 40-60 bar, for example 50 bar). The nutritional composition can also be sterilized. Sterilization can, for example, involve a pre-heating step (which can, e.g., be carried out at 70-100°C, preferably 85-95°C, e.g. for 2-5 minutes), a heating step (which can, e.g., be carried out at 130-145°C, preferably 139-141°C, e.g. for 6-15 seconds) and an additional homogenization step (which can, e.g., be carried out at a pressure of 40-200 bar, e.g. at < 90°C).

The nutritional composition can, for example, be prepared by a process which comprises the following steps:
i. a first step, wherein water is provided and heated to a temperature of 50-70°C;
ii. a second step wherein, optionally, a carbohydrate component - or a fraction thereof - and, optionally, a stabilizer, such as MCC, is added;
iii. a third step, wherein the protein component is added;
iv. a fourth step, wherein the lipid component is added, preferably at an elevated temperature of 80-90°C, preferably in combination with a (further) emulsifier and/or stabilizer, such as soy lecithin and/or diacetyl tartaric acid glycerides (e.g. DATEM), and/or (distilled) monoglycerides;
v. a fifth step, wherein vitamins and, optionally, minerals and, optionally, the remaining fraction of the carbohydrate component and, optionally, any other powder ingredient, e.g. fibres, aroma, and other additives, are added; at any time before, during or after the fifth step, the mixture is allowed to cool to 55-65°C;
vi. a sixth step, wherein the pH of the mixture is adjusted to 6-9, preferably 7-8, e.g. 7;
vii. a seventh step, wherein the mixture is homogenized, e.g. at 60-65°C at a pressure of 100/50 bar;
viii. an eighth step, wherein the mixture is sterilized; the eighth step may comprise (8.a) a pre-heating step, (8.b) a heating step and (8.c) an additional homogenization step.

In an alternative embodiment, all or a part of the additional ingredients, such as aroma, may be added already between the third or fourth step.

Preferably, step 8 is divided into:
a) a step 8.a, wherein the mixture is pre-heated to 70-100°C, preferably 85-95°C, e.g. for 2-5 minutes;
b) a step 8.b, wherein the mixture is heated to 130-145°C, preferably to 139-141°C, e.g. for 6-15 seconds; and
c) a step 8.c, wherein the mixture is homogenized at a pressure of 40-200bar; preferably step 8.c is carried out at less than 90°C.

### Dose unit and daily dose

The nutritional compositions herein are typically provided in a dose unit.

A dose unit herein refers to 100-200mL, preferably 100-150mL, preferably provided in package such as a bottle, tetra brick or bag.

Such a dose unit provides 300-500kcal, preferably 350-450kcal.

Such a dose unit provides 14-40g, preferably 15-30g, more preferably 18-22g of protein, such as 20g of protein.

An exemplary dose unit provides 400kcal and 20g of protein in 125mL.

An exemplary daily dose for complete nutrition of, e.g., 1500-2500kcal of the nutritional compositions herein may be provided by 3-8 dose units, preferably by 5 dose units. For example, a daily dose for complete nutrition may be provided by 5 dose units each providing 350-450kcal.

A typical daily dose for supplemental nutrition of, e.g., 300-900kcal of the nutritional compositions herein may be provided by 1-3 dose units, preferably by 1 or 2 dose units, for example by 1 or 2 dose units each providing 350-450kcal.

### EXAMPLES

The nutritional composition according to Table 1 was prepared by mixing and homogenization.

Surprisingly, high caloric densities could be reached by adding collagen hydrolysate in sufficient amounts to milk protein.

The Example (Table 1) further shows that with inclusion of sufficient amounts of collagen hydrolysate very high energy densities of protein could be reached in a formula that provides very high caloric density (3.2 kcal/mL).

The inventors further observed that fouling during heat treatment could be reduced / prevented when sufficient amounts of collagen hydrolysate were used in the protein component. Advantages observed with the nutritional composition according to Table 1 include an improved stability against subsequent UHT (no fouling), an improved viscosity and an improved shelf life (at least 6 months).

With the nutritional composition, a drinkable viscosity of a nutritionally complete composition can be provided in spite of a high protein and energy content.

The inventors further observed that bitterness of the product could be reduced by increasing wt% of collagen hydrolysate in the protein component. Therefore, the nutritional composition will result in a better patient compliance.

### Trials

The nutritional composition according to Table 1 (herein referred to as "test drink") was given to 20 subjects with an indication for supplemental nutrition of approx. 400kcal per day. Subjects belong to the age group of 65 and older. The dosage was one bottle of 125mL per day for seven consecutive days. One such bottle provides 400kcal, including 20g protein (20 EN%), 20g fat (45 EN%) and 35g carbohydrates (35 EN%). 14 EN% is provided by PUFA.

Adverse effects were documented. Gastrointestinal tolerance parameters were documented. Moreover, (protein) energy intake, compliance (in particular time until full consumption of the test drink and/or amount consumed within 1 hour) and palatability were documented.

### Trial 1

8 males and 12 females participated in the study. The test drink was administered in a single dose (125mL).

All 20 subjects completed the 7 days of intervention. In total, 96% of the prescribed test drinks were consumed. All study participants ingested the test drink within one hour.

Gastrointestinal (GI) symptoms were comparable at baseline and at the end of the 7 day supplementation period. No severe GI symptoms were observed. 11 of the 20 subjects did not report any GI symptoms during the entire study.

The palatability assessment showed a very high acceptance of the test drink. The majority of the subjects rated smell, taste and appearance as 'good' at the start and at the end of the study. No rating of poor or unacceptable was observed. Furthermore, there was no appearance of taste fatigue, even though only one flavor was provided throughout the study.

The results of the palatability assessment (overall opininon) are as follows:

| **Palatability (overall opinion)** | | **Excellent** | **Good** | **Fair** | **Poor** | **Unacceptable** |
|---|---|---|---|---|---|---|
| **Observations** (Number of study participants) | Study day 1 | 3 | 13 | 4 | 0 | 0 |
| | Study day 7 | 3 | 10 | 7 | 0 | 0 |

The results of the taste assessment are as follows:

| **Taste** | | **Excellent** | **Good** | **Fair** | **Poor** | **Unacceptable** |
|---|---|---|---|---|---|---|
| **Observations** (Number of study participants) | Study day 1 | 4 | 12 | 4 | 0 | 0 |
| | Study day 7 | 2 | 13 | 5 | 0 | 0 |

In conclusion, the test drink administered in a single dose was well-accepted. The test drink showed good tolerability and palatability resulting in excellent compliance.

### Trial 2

9 males and 11 females participated in the study. The test drink was administered in three doses (about 40mL each).

All 20 subjects completed the 7 days of intervention. In total, 95% of the prescribed test drinks were consumed. All study participants ingested the test drink immediately after handout.

Gastrointestinal (GI) symptoms were comparable at baseline and at the end of the 7 day supplementation period. No severe GI symptoms were observed. 13 of the 20 subjects did not report any GI symptoms during the entire study.

Palatability was rated as excellent or good by the majority of the subjects. No rating of poor or unacceptable was observed. Furthermore, there was no appearance of taste fatigue, even though only one flavor was provided throughout the study. Perception of sweetness was rated as just right by the majority of the subjects.

The results of the palatability assessment (overall opininon) are as follows:

| **Palatability (overall opinion)** | | **Excellent** | **Good** | **Fair** | **Poor** | **Unacceptable** |
|---|---|---|---|---|---|---|
| **Observations** (Number of study participants) | Study day 1 | 3 | 13 | 4 | 0 | 0 |
| | Study day 7 | 1 | 16 | 3 | 0 | 0 |

The results of the sweetness assessment are as follows:

| **Sweetness** | | **Extremely sweet** | **Highly Sweet** | **Just right** | **Slightly sweet** | **Not sweet at all** |
|---|---|---|---|---|---|---|
| **Observations** (Number of study participants) | Study day 1 | 0 | 3 | 12 | 4 | 0 |
| | Study day 7 | 0 | 2 | 17 | 1 | 0 |

In conclusion, the test drink administered in three doses was well-accepted. The test drink showed good tolerability and palatability resulting in excellent compliance.

### METHODS

### Determination of molecular weight of the hydrolysed collagen by GPC/HPLC

Equipment: GPV/HPLC with UV detector operating at 214 nm. Column: TSK 2000 SW XL (Toshoh Biosience GmbH). Isocratic elution using 400 mmol/l sodium phosphate buffer (pH 5.3). Calibration by means of well-defined Type I-collagen fragments (FILK, Freiburg, Germany). The collagen hydrolysate used in the examples had an average molecular weight of 2kDa. In general, the skilled person is well aware of molecular weight determination via GPC. Another suitable method for determining the M_{w} of small macromolecules such as the hydrolysates described herein is MALDI-MS.

**TABLE 1: Examples**

| | |
|---|---|
| Protein: | 20 EN% |
| Collagen hydrolysate 80% | 16 g/100mL |
| Milk protein 20% (Refit 10,8% / MPC-80 9.2%) | |
| glycine* | 3.42 g/100mL |
| arginine* | 1.05 g/100mL |
| tryptophan* | 0.05 g/100mL |
| proline* | 2.38 g/100mL |
| cysteine* | 0.03 g/100mL |
| Fat: | 45 EN% |
| Rapeseed oil | 16 g/100mL |
| of which SFA | 3 EN% |
| | 1.1 g/100mL |
| of which MUFA | 28 EN% |
| | 9.9 g/100mL |
| of which PUFA | 14 EN% |
| | 5.0 g/100mL |
| CHO | 35 EN% |
| | 28 g/100mL |
| Caloric density | 3.2 kcal/mL |
| Water | 56 mL/100mL |
| FSMP balanced ** | yes |
| Vitamin D3 | 8 µg/100mL |
| Vitamin E | 5.67 mg/100mL (alpha- tocopherol equivalents (alpha- TE)) |

| | |
|---|---|
| * Bound in collagen hydrolysate or milk protein ** Nutritionally complete in vitamins and minerals | |

**TABLE 2: Vitamins**

| | Minimum per 100kcal | Maximum per 100kcal |
|---|---|---|
| Vitamin A (µg RE) | 35 | 180 |
| Vitamin D (µg) | 0,5 | 3 |
| Vitamin K (µg) | 3,5 | 20 |
| Vitamin C (mg) | 2,2 | 22 |
| Thiamin (mg) | 0,06 | 0,5 |
| Riboflavin (mg) | 0,08 | 0,5 |
| Vitamin B6 (mg) | 0,08 | 0,5 |
| Niacin (mg EN) | 0,9 | 3 |
| Folic acid (µg) | 10 | 50 |
| Vitamin B12 (µg) | 0,07 | 0,7 |
| Pantothenic acid (mg) | 0,15 | 1,5 |
| Biotin (µg) | 0,75 | 7,5 |
| Vitamin E (mg α-TE) | 0,5 | 3 |

**TABLE 3: Minerals**

| | Minimum per 100kcal | Maximum per 100kcal |
|---|---|---|
| Sodium (mg) | 30 | 175 |
| Chloride (mg) | 30 | 175 |
| Potassium (mg) | 80 | 295 |
| Calcium (mg) | 35 | 250 |
| Phosphorus (mg) | 30 | 80 |
| Magnesium (mg) | 7,5 | 25 |
| Iron (mg) | 0,5 | 2,0 |
| Zinc (mg) | 0,5 | 1,5 |
| Copper (µg) | 60 | 500 |
| Iodine (µg) | 6,5 | 35 |
| Selenium (µg) | 2,5 | 10 |
| Manganese (mg) | 0,05 | 0,5 |
| Chromium (µg) | 1,25 | 15 |
| Molybdenum (µg) | 3,5 | 18 |
| Fluoride (mg) | - | 0,2 |

## Claims

1. PUFA, vitamin E, vitamin D and the protein bound amino acids glycine, arginine and tryptophan as active ingredients for use in the prevention or treatment of malnutrition associated with COPD, for use in the prevention or treatment of malnutrition associated with neurological disease or neurological disorders and for use in prevention or treatment of malnutrition during wound healing,
wherein an effective amount of said active ingredients is administered in the form of a nutritionally complete liquid nutritional composition comprising
a) a lipid component providing 40-50 EN% based on the total energy of the nutritional composition, wherein 12-16 EN% based on the total energy of the nutritional composition is provided by PUFA,
b) 4.0-8.0 mg/100mL alpha-tocopherol equivalents (alpha-TE) vitamin E,
c) 5.0-12.0 µg/100mL vitamin D,
d) a protein component, wherein the protein component consists of collagen hydrolysate as a first protein source and milk protein as a second protein source, wherein collagen hydrolysate is present at a concentration from 70-90 wt% based on the total weight of the protein component and the milk protein is present at a concentration from 10-30 wt% based on the total weight of the protein component and wherein the protein component provides 15-40 EN% based on the total energy of the nutritional composition, wherein the collagen hydrolysate has an average molecular weight M_{w} of from 1000 to 6000 Da, wherein the average molecular weight M_{w} is determined as set forth in the description,
e) 2.5-4.5 g/100mL protein bound glycine,
f) 0.5-1.5 g/100mL protein bound arginine,
g) at least 0.02 g/100mL protein bound tryptophan, and
h) wherein the composition has a caloric density of at least 2.0 kcal/ml.

2. Composition for use according to any of the preceding claims, wherein the lipid component comprises rapeseed oil and/or canola oil, preferably rapeseed oil, also preferably canola oil.

3. Composition for use according to any of the preceding claims, wherein the nutritional composition comprises at least 10 wt% of protein based on the total weight of the nutritional composition.

4. Composition for use according to any of the preceding claims, wherein the nutritional composition comprises at least 14 g of protein per 100 mL of the nutritional composition.

5. Composition for use according to any of the preceding claims, wherein the nutritional composition further comprises 1.5-4.0 g/100mL proline.

6. Composition for use according to any of the preceding claims, wherein the nutritional composition further comprises at least 0.01 g/100mL cysteine.

7. Composition for use according to any of the preceding claims, wherein the nutritional composition further comprises 50-300 mg/100mL calcium.

8. Composition for use according to any of the preceding claims, wherein the nutritional composition is an oil-in-water (O/W) emulsion.

9. Composition for use according to any of the preceding claims, wherein the patients have a BMI of < 18.5 kg/m2.

10. Composition for use according to any of the preceding claims, wherein the patients have an unintentional weight loss and either
(i) a reduced BMI of < 20 kg/m2 in subjects younger than 70 years or < 22 kg/m2 in subjects older than 70 years or
(ii) a low fat free mass index (FFMI) < 15 kg/m2 in females or < 17 kg/m2 in males, wherein loss is defined as either > 10% of habitual weight indefinite of time, or >5% over 3 months.

11. Composition for use according to any of the preceding claims, wherein the nutritional composition is provided in a dose unit of 100-200 mL or in a dose unit providing 300-500kcal or in a dose unit providing 15-30g of protein.

12. Composition for use according to any of the preceding claims, wherein the nutritional composition is administered in a daily dose of 1500-2500kcal or in a daily dose of 300-900kcal or in a daily dose of 75-125g of protein or in a daily dose of 15-45g of protein.

13. Nutritionally complete liquid nutritional composition comprising
a) a lipid component providing 40-50 EN% based on the total energy of the nutritional composition, wherein 12-16 EN% based on the total energy of the nutritional composition is provided by PUFA,
b) 4.0-8.0 mg/100mL alpha-tocopherol equivalents (alpha-TE) vitamin E,
c) 5.0-12.0 µg/100mL vitamin D,
d) a protein component, wherein the protein component consists of collagen hydrolysate as a first protein source and milk protein as a second protein source, wherein collagen hydrolysate is present at a concentration from 70-90 wt% based on the total weight of the protein component and the milk protein is present at a concentration from 10-30 wt% based on the total weight of the protein component and wherein the protein component provides 15-40 EN% based on the total energy of the nutritional composition, wherein the collagen hydrolysate has an average molecular weight M_{w} of from 1000 to 6000 Da, wherein the average molecular weight M_{w} is determined as set forth in the description,
e) 2.5-4.5 g/100mL protein bound glycine,
f) 0.5-1.5 g/100mL protein bound arginine,
g) at least 0.02 g/100mL protein bound tryptophan, and
h) wherein the composition has a caloric density of at least 2.0 kcal/ml.

## Patentansprüche

1. PUFA, Vitamin E, Vitamin D und die proteingebundenen Aminosäuren Glycin, Arginin und Tryptophan als Wirkstoffe zur Verwendung bei der Vorbeugung oder Behandlung von Mangelernährung im Zusammenhang mit COPD, zur Verwendung bei der Vorbeugung oder Behandlung von Mangelernährung im Zusammenhang mit neurologischen Krankheiten oder neurologischen Störungen und zur Verwendung bei der Vorbeugung oder Behandlung von Mangelernährung während der Wundheilung,
wobei eine wirksame Menge der Wirkstoffe in Form einer diätetisch vollständigen flüssigen Nährstoffzusammensetzung verabreicht wird, die Folgendes umfasst:
a) eine Lipidkomponente, die 40-50 EN % basierend auf der Gesamtenergie der Nährstoffzusammensetzung bereitstellt, wobei 12-16 EN % basierend auf der Gesamtenergie der Nährstoffzusammensetzung durch PUFA bereitgestellt werden,
b) 4,0-8,0 mg/100 mL alpha-Tocopherol-Äquivalente (alpha-TE) Vitamin E,
c) 5,0-12,0 µg/100 mL Vitamin D,
d) eine Proteinkomponente, wobei die Proteinkomponente aus Kollagenhydrolysat als erster Proteinquelle und Milchprotein als zweiter Proteinquelle besteht, wobei das Kollagenhydrolysat in einer Konzentration von 70-90 Gew.-% basierend auf dem Gesamtgewicht der Proteinkomponente vorhanden ist und das Milchprotein in einer Konzentration von 10-30 Gew.-% basierend auf dem Gesamtgewicht der Proteinkomponente vorhanden ist und wobei die Proteinkomponente 15-40 EN % basierend auf der Gesamtenergie der Nährstoffzusammensetzung bereitstellt, wobei das Kollagenhydrolysat ein durchschnittliches Molekulargewicht M_{w} von 1000 bis 6000 Da aufweist, wobei das durchschnittliche Molekulargewicht M_{w} wie in der Beschreibung dargestellt bestimmt wird,
e) 2,5-4,5 g/100 mL proteingebundenes Glycin,
f) 0,5-1,5 g/100 mL proteingebundenes Arginin,
g) mindestens 0,02 g/100 mL proteingebundenes Tryptophan und
h) wobei die Zusammensetzung eine Kaloriendichte von mindestens 2,0 kcal/mL aufweist.

2. Zusammensetzung zur Verwendung nach einem der vorhergehenden Ansprüche, wobei die Lipidkomponente Rapsöl und/oder Canolaöl umfasst, vorzugsweise Rapsöl, ebenfalls vorzugsweise Canolaöl.

3. Zusammensetzung zur Verwendung nach einem der vorhergehenden Ansprüche, wobei die Nährstoffzusammensetzung mindestens 10 Gew.-% Protein basierend auf dem Gesamtgewicht der Nährstoffzusammensetzung umfasst.

4. Zusammensetzung zur Verwendung nach einem der vorhergehenden Ansprüche, wobei die Nährstoffzusammensetzung mindestens 14 g Protein pro 100 mL der Nährstoffzusammensetzung umfasst.

5. Zusammensetzung zur Verwendung nach einem der vorhergehenden Ansprüche, wobei die Nährstoffzusammensetzung ferner 1,5-4,0 g/100 mL Prolin umfasst.

6. Zusammensetzung zur Verwendung nach einem der vorhergehenden Ansprüche, wobei die Nährstoffzusammensetzung ferner mindestens 0,01 g/100 mL Cystein umfasst.

7. Zusammensetzung zur Verwendung nach einem der vorhergehenden Ansprüche, wobei die Nährstoffzusammensetzung ferner 50-300 mg/100 mL Calcium umfasst.

8. Zusammensetzung zur Verwendung nach einem der vorhergehenden Ansprüche, wobei die Nährstoffzusammensetzung eine Öl-in-Wasser (O/W)-Emulsion ist.

9. Zusammensetzung zur Verwendung nach einem der vorhergehenden Ansprüche, wobei die Patienten einen BMI von < 18,5 kg/m² aufweisen.

10. Zusammensetzung zur Verwendung nach einem der vorhergehenden Ansprüche, wobei die Patienten einen ungewollten Gewichtsverlust aufweisen und entweder
(i) einen reduzierten BMI von < 20 kg/m² bei Individuen, die jünger als 70 Jahre sind, oder < 22 kg/m² bei Individuen, die älter als 70 Jahre sind, oder
(ii) einen niedrigen fettfreien Massenindex (FFMI) < 15 kg/m² bei Frauen oder < 17 kg/m² bei Männern,
wobei Verlust definiert ist als entweder > 10 % des gewohnten Gewichts auf unbestimmte Zeit oder > 5 % über 3 Monate.

11. Zusammensetzung zum Verwenden nach einem der vorhergehenden Ansprüche, wobei die Nährstoffzusammensetzung in einer Dosiseinheit von 100-200 mL oder in einer Dosiseinheit, die 300-500 kcal bereitstellt oder in einer Dosiseinheit, die 15-30 g Protein bereitstellt, bereitgestellt wird.

12. Zusammensetzung zum Verwenden nach einem der vorhergehenden Ansprüche, wobei die Nährstoffzusammensetzung in einer Tagesdosis von 1500-2500 kcal oder in einer Tagesdosis von 300-900 kcal oder in einer Tagesdosis von 75-125 g Protein oder in einer Tagesdosis von 15-45 g Protein verabreicht wird.

13. Diätetisch vollständige, flüssige Nährstoffzusammensetzung, die Folgendes umfasst:
a) eine Lipidkomponente, die 40-50 EN % basierend auf der Gesamtenergie der Nährstoffzusammensetzung bereitstellt, wobei 12-16 EN % basierend auf der Gesamtenergie der Nährstoffzusammensetzung durch PUFA bereitgestellt werden,
b) 4,0-8,0 mg/100 mL alpha-Tocopherol-Äquivalente (alpha-TE) Vitamin E,
c) 5,0-12,0 µg/100 mL Vitamin D,
d) eine Proteinkomponente, wobei die Proteinkomponente aus Kollagenhydrolysat als erster Proteinquelle und Milchprotein als zweiter Proteinquelle besteht, wobei das Kollagenhydrolysat in einer Konzentration von 70-90 Gew.-% basierend auf dem Gesamtgewicht der Proteinkomponente vorhanden ist und das Milchprotein in einer Konzentration von 10-30 Gew.-% basierend auf dem Gesamtgewicht der Proteinkomponente vorhanden ist und wobei die Proteinkomponente 15-40 EN % basierend auf der Gesamtenergie der Nährstoffzusammensetzung bereitstellt, wobei das Kollagenhydrolysat ein durchschnittliches Molekulargewicht M_{w} von 1000 bis 6000 Da aufweist, wobei das durchschnittliche Molekulargewicht M_{w} wie in der Beschreibung dargestellt bestimmt wird,
e) 2,5-4,5 g/100 mL proteingebundenes Glycin,
f) 0,5-1,5 g/100 mL proteingebundenes Arginin,
g) mindestens 0,02 g/100 mL proteingebundenes Tryptophan und
h) wobei die Zusammensetzung eine Kaloriendichte von mindestens 2,0 kcal/mL aufweist.

## Revendications

1. AGPI, vitamine E, vitamine D et les acides aminés liés à des protéines glycine, arginine et tryptophane en tant que principes actifs destinés à être utilisés dans la prévention ou le traitement de malnutrition associée à la BPCO, destinés à être utilisés dans la prévention ou le traitement de malnutrition associée à une maladie neurologique ou à des troubles neurologiques et destinés à être utilisés dans la prévention ou le traitement de malnutrition pendant la cicatrisation de plaies,
une quantité efficace desdits principes actifs étant administrée sous la forme d'une composition nutritionnelle liquide nutritionnellement complète comprenant
a) un composant lipidique fournissant 40 à 50 % de l'énergie sur la base de l'énergie totale de la composition nutritionnelle, 12 à 16% de l'énergie sur la base de l'énergie totale de la composition nutritionnelle étant fournie par des AGPI,
b) 4,0 à 8,0 mg/100 ml d'équivalents alpha-tocophérol (alpha-TE) de vitamine E,
c) 5,0 à 12,0 µg/100 ml de vitamine D,
d) un composant protéique, le composant protéique étant constitué d'hydrolysat de collagène en tant que première source de protéines et de protéines de lait en tant que seconde source de protéines, l'hydrolysat de collagène étant présent en une concentration allant de 70 à 90 % en poids sur la base du poids total du composant protéique et les protéines de lait étant présentes en une concentration allant de 10 à 30 % en poids sur la base du poids total du composant protéique et le composant protéique fournissant 15 à 40 % de l'énergie sur la base de l'énergie totale de la composition nutritionnelle, l'hydrolysat de collagène ayant un poids moléculaire moyen M_{w} allant de 1000 à 6000 Da, le poids moléculaire moyen M_{w} étant déterminé comme indiqué dans la description,
e) 2,5 à 4,5 g/100 ml de glycine liée à une protéine,
f) 0,5 à 1,5 g/100 ml d'arginine liée à une protéine,
g) au moins 0,02 g/100 ml de tryptophane lié à une protéine et
h) la composition ayant une densité calorique d'au moins 2,0 kcal/ml.

2. Composition destinée à être utilisée selon l'une quelconque des revendications précédentes, dans laquelle le composant lipidique comprend de l'huile de colza et/ou de l'huile de canola, de préférence de l'huile de colza, également de préférence de l'huile de canola.

3. Composition destinée à être utilisée selon l'une quelconque des revendications précédentes, la composition nutritionnelle comprenant au moins 10 % en poids de protéines sur la base du poids total de la composition nutritionnelle.

4. Composition destinée à être utilisée selon l'une quelconque des revendications précédentes, la composition nutritionnelle comprenant au moins 14 g de protéines pour 100 ml de la composition nutritionnelle.

5. Composition destinée à être utilisée selon l'une quelconque des revendications précédentes, la composition nutritionnelle comprenant en outre 1,5 à 4,0 g/100 ml de proline.

6. Composition destinée à être utilisée selon l'une quelconque des revendications précédentes, la composition nutritionnelle comprenant en outre au moins 0,01 g/100 ml de cystéine.

7. Composition destinée à être utilisée selon l'une quelconque des revendications précédentes, la composition nutritionnelle comprenant en outre 50 à 300 mg/100 ml de calcium.

8. Composition destinée à être utilisée selon l'une quelconque des revendications précédentes, la composition nutritionnelle étant une émulsion huile dans eau (H/E).

9. Composition destinée à être utilisée selon l'une quelconque des revendications précédentes, les patients ayant un IMC < 18,5 kg/m2.

10. Composition destinée à être utilisée selon l'une quelconque des revendications précédentes, les patients ayant une perte de poids involontaire et soit
(i) un IMC réduit < 20 kg/m2 chez les sujets de moins de 70 ans ou < 22 kg/m2 chez les sujets de plus de 70 ans soit
(ii) un faible indice de masse maigre (IMM) < 15 kg/m2 chez les femmes ou < 17 kg/m2 chez les hommes,
la perte étant définie comme soit > 10 % du poids habituel sur une durée indéterminée soit > 5 % sur 3 mois.

11. Composition destinée à être utilisée selon l'une quelconque des revendications précédentes, la composition nutritionnelle étant fournie en une dose unitaire de 100 à 200 ml ou en une dose unitaire fournissant 300 à 500 kcal ou en une dose unitaire fournissant 15 à 30 g de protéines.

12. Composition destinée à être utilisée selon l'une quelconque des revendications précédentes, la composition nutritionnelle étant administrée en une dose journalière de 1500 à 2500 kcal ou en une dose journalière de 300 à 900 kcal ou en une dose journalière de 75 à 125 g de protéines ou en une dose journalière de 15 à 45 g de protéines.

13. Composition nutritionnelle liquide nutritionnellement complète comprenant
a) un composant lipidique fournissant 40 à 50 % de l'énergie sur la base de l'énergie totale de la composition nutritionnelle, 12 à 16% de l'énergie sur la base de l'énergie totale de la composition nutritionnelle étant fournie par des AGPI,
b) 4,0 à 8,0 mg/100 ml d'équivalents alpha-tocophérol (alpha-TE) de vitamine E,
c) 5,0 à 12,0 µg/100 ml de vitamine D,
d) un composant protéique, le composant protéique étant constitué d'hydrolysat de collagène en tant que première source de protéines et de protéines de lait en tant que seconde source de protéines, l'hydrolysat de collagène étant présent en une concentration allant de 70 à 90 % en poids sur la base du poids total du composant protéique et les protéines de lait étant présentes en une concentration allant de 10 à 30 % en poids sur la base du poids total du composant protéique et le composant protéique fournissant 15 à 40 % de l'énergie sur la base de l'énergie totale de la composition nutritionnelle, l'hydrolysat de collagène ayant un poids moléculaire moyen M_{w} allant de 1000 à 6000 Da, le poids moléculaire moyen M_{w} étant déterminé comme indiqué dans la description,
e) 2,5 à 4,5 g/100 ml de glycine liée à une protéine,
f) 0,5 à 1,5 g/100 ml d'arginine liée à une protéine,
g) au moins 0,02 g/100 ml de tryptophane lié à une protéine et
h) la composition ayant une densité calorique d'au moins 2,0 kcal/ml.
